# FASCICULE DE BREVET EUROPEEN

(11) **EP 2 377 573 B1**
(45) Date de publication et mention de la délivrance du brevet: **09.05.2012**
(21) Numéro de dépôt: 11152309.8
(22) Date de dépôt: 27.01.2011
(51) Int. Cl.: A61N 1/375, H05K 5/06

(54) **Procédé de réalisation d'une traversée électrique dans la paroi métallique d'un boîtier, notamment de dispositif médical actif, et dispositif pourvu d'une telle traversée**
Verfahren zur Herstellung einer elektrischen Kabeldurchführung durch eine Metallwand eines Gehäuses, insbesondere einer aktiven medizinischen Vorrichtung, und mit einer solchen Durchführung ausgestattete Vorrichtung
Method for manufacturing an electric cross-piece in the metal wall of a box, in particular of an active medical device, and device provided with such a cross-piece

(30) Priorité: 22.03.2010 FR 1052037
(43) Date de publication de la demande: 19.10.2011
(73) Titulaire: Sorin CRM SAS, 92143 Clamart Cedex (FR)
(72) Inventeur: Boutaud, Bertrand, 75014, PARIS (FR); Viatgé, Hélène, 92120, MONTROUGE (FR)
(74) Mandataire: Dupuis-Latour, Dominique

(56) Documents cités:
- WO-A2-2008/067519
- US-A- 5 166 097
- US-A- 5 322 816

## Description

L'invention concerne la technologie de fabrication des "dispositifs médicaux implantables actifs" tels que définis par la directive 90/385/CEE du 20 juin 1990 du Conseil des communautés européennes.

Cette définition inclut notamment les appareils chargés de surveiller en continu l'activité cardiaque et délivrer si nécessaire au coeur des impulsions électriques de stimulation, de resynchronisation et/ou de défibrillation en cas de trouble du rythme détecté par le dispositif. Elle inclut aussi les appareils neurologiques, les implants cochléaires, etc., ainsi que les dispositifs de mesure de pH ou autres paramètres intracorporels.

Ces dispositifs comportent un générateur constitué d'un boîtier métallique, généralement en titane, sur lequel est montée une tête de connecteur. Le connecteur est pourvu de logements permettant de raccorder mécaniquement et électriquement au boîtier du générateur une ou plusieurs sondes portant à leur extrémité distale diverses électrodes de recueil, de stimulation et de défibrillation.

La liaison du connecteur aux divers circuits électroniques enfermés dans le boîtier implique la réalisation de plusieurs traversées de ce boîtier, avec pour chacune un picot destiné d'une part à être relié à une borne correspondante de la tête de connecteur par son extrémité émergeant sur la face supérieure du boîtier (côté externe), et d'autre part à être relié aux circuits électroniques à son extrémité opposée (côté interne) débouchant dans le volume intérieur du boîtier où ses trouvent ces circuits.

Une telle traversée est par exemple décrite dans le EP 1 177 815 A1 (ELA Medical).

Outre les picots de liaison à la tête de connecteur, d'autres traversées peuvent être également prévues, par exemple pour assurer une liaison avec une électrode de surface disposée sur la face externe du boîtier, ou encore au niveau d'un capteur intégré à la sonde du dispositif, comme cela est décrit par le WO 2008/067519 A2.

Ces traversées peut être également rencontrées dans des sous-composants des dispositifs médicaux tels que les batteries et les condensateurs.

En tout état de cause, la traversée doit isoler électriquement le conducteur traversant (picot ou autre) du boîtier métallique, et elle doit être parfaitement hermétique pour éviter toute pénétration de fluides à l'intérieur du boîtier, et ceci pendant toute la durée de vie du dispositif implanté, typiquement pendant une dizaine d'années. Cette traversée est donc un des éléments clefs des boîtiers des défibrillateurs et des stimulateurs, car elle assure une double fonction de passage du courant électrique et d'étanchéité du boîtier.

Pour l'isolement électrique, on utilise actuellement des matériaux diélectriques tels que céramique ou verre, qui permettent en outre la réalisation de liaisons mécaniques très résistantes avec le métal du boîtier. A cet effet, le matériau isolant de la traversée est cerclé d'une collerette métallique permettant le soudage de la traversée au boîtier (ou au demi-boîtier) du dispositif. Quant aux liaisons entre les picots et le matériau isolant, elles sont rendues hermétiques par des brasures à l'or qui assurent à cet endroit une étanchéité et une isolation parfaites.

La complexité de la réalisation de ces traversées et l'utilisation de composants et de technologies très spécifiques expliquent en partie le prix élevé des traversées, qui peuvent représenter jusqu'à 10 % du prix d'un dispositif. En outre, la tendance actuelle à la multiplication du nombre d'électrodes (avec les dispositifs "multisite" notamment) implique une augmentation corrélative du nombre de contacts sur le connecteur, et donc de celui picots, ce qui renchérit d'autant le coût de réalisation de la traversée.

D'autres difficultés subsistent. En particulier, du fait que la traversée devra être intégrée au boîtier, la fermeture hermétique de celui-ci, qui est en titane, est généralement réalisée par soudage laser, ce qui rend cette opération complexe du fait de l'introduction de jeux à la fois dans le sens vertical et dans le sens horizontal, jeux parfois trop importants pour que le soudage laser soit efficace. D'autre part, avec l'intégration au boîtier, les traversées constituent en elles-mêmes des structures très hétérogènes qui subissent des contraintes thermomécaniques importantes au moment du soudage laser, et qui sont donc régulièrement le lieu de défauts de fiabilité.

Les traversées représentent donc un composant très critique du boîtier.

D'autre part, du point de vue de l'encombrement, les traversées imposent un regroupement de tous les fils électriques sur la zone de la traversée.

Comme ces fils électriques sont amenés à être reliés au connecteur en des points relativement éloignés, il est nécessaire d'imposer des courbures relativement importantes aux picots électriques au débouché de la traversée, afin de diriger ces picots vers les points de contact adéquats de la tête de connecteur.

Enfin, la place relativement importante prise par la traversée à l'intérieur et à l'extérieur du boîtier limite sérieusement les possibilités de miniaturisation au moment de la conception du dispositif.

En résumé, bien que les traversées telles qu'elles existent aujourd'hui remplissent parfaitement leurs fonctions (d'une part d'herméticité et d'autre part de transfert de l'information électrique), elles représentent une part trop importante dans le coût de fabrication des implants médicaux, une contrainte à la conception de ceux-ci, une limite à la miniaturisation et une source non négligeable de défauts de fiabilité lors de l'assemblage du boitier, difficultés dont on n'a pas à ce jour réussi à s'affranchir.

Diverses techniques alternatives ont été proposées, toutes relativement complexes et non dénuées d'inconvénients.

Ainsi, le US 2007/0112396 A1 décrit une technique de réalisation d'une traversée pour un implant médical tel qu'une prothèse cochléaire. La traversée est réalisée en dopant localement du silicium pour le rendre conducteur sur certains chemins seulement, en lieu et place d'un conducteur (picot) s'étendant de part en part de la traversée. La difficulté réside dans l'absence d'isolement physique de la zone de conduction électrique, dans la mesure où l'interface entre le silicium dopé et le silicium non dopé ne peut pas être considérée comme totalement isolante. En outre, cette technique impose le report sur le boîtier du dispositif de la plaquette de silicium sur laquelle a été réalisée la traversée, avec nécessité d'un brasage silicium/titane présentant à peu près les mêmes inconvénients et difficultés de réalisation que les brasages céramique/titane évoqués plus haut.

Une autre proposition est formulée dans le US 2007/0060969 A1, qui propose de réaliser la traversée en empilant un certain nombre de couches de céramique crue pourvues de vias décalés, interconnectés par une colle conductrice appliquée entre les diverses couches. Cette technique présente cependant les mêmes inconvénients que la précédente, à savoir l'absence de séparation physique entre isolant et conducteur (bien que dans ce cas les matériaux soient différents), et la nécessité de reporter la traversée céramique sur le boîtier en titane avec réalisation d'un brasage hermétique. De plus, le coût de réalisation est relativement élevé du fait d'opérations relativement complexes et de l'emploi de céramique et de matériaux conducteurs en platine/iridium, voire en or.

Dans des domaines (aéronautique notamment) autres que celui des implants médicaux d'autres techniques encore ont été proposées, décrites par exemple dans les US 5 166 097 A et US 5 322 816 A. Mais il s'agit toujours de former des traversées dans une plaquette de silicium, plaquette qu'il sera nécessaire de reporter ultérieurement sur le boîtier métallique avec toutes les contraintes technologiques qu'implique cette opération.

L'invention propose d'assurer la continuité électrique dans la région de la traversée entre les deux côtés du boîtier en s'affranchissant de toute pièce intermédiaire tout en assurant une parfaite herméticité et un isolement électrique avec le boîtier. L'absence de composant supplémentaire permettra notamment de s'affranchir des difficultés soulevées lors du soudage laser du fait des jeux verticaux et horizontaux dus à ce composant supplémentaire.

Le point de départ de l'invention repose sur la création dans la masse de la paroi du boîtier, à l'endroit de la traversée, d'une zone dédiée à la conduction électrique, mais isolée physiquement et donc électriquement du reste du corps de boîtier.

Pour que cette zone isolée reste solidaire du reste du boîtier et pour empêcher le passage des fluides au travers de l'intervalle d'isolement définissant cette zone, une couche isolante est créée ou ajoutée sur le boîtier, par exemple par oxydation du titane ou dépôt d'une couche de dioxyde de silicium.

Plus précisément, le procédé de l'invention consiste, pour la réalisation d'une traversée hermétique et électriquement isolante permettant le passage d'une liaison électrique au travers de la paroi du dispositif, en une succession d'étapes de : a) formation d'une couche électriquement isolante sur chacune des faces externe et interne de la paroi métallique électriquement conductrice du dispositif ; b) côté interne de la paroi, formation d'une gorge non traversante de contour fermé délimitant dans la paroi un îlot métallique physiquement et électriquement isolé du reste de la paroi, par creusement de la totalité de l'épaisseur de la couche interne électriquement isolante et de celle de la paroi, en laissant intacte l'épaisseur de la couche externe électriquement isolante de manière à faire supporter mécaniquement l'îlot métallique par cette couche externe ; et c) mise à nu côté externe, et respectivement côté interne, d'une plage de contact électrique à l'îlot métallique, par enlèvement localisé de matière sur l'épaisseur de la couche externe, respectivement interne, électriquement isolante.

La couche électriquement isolante formée à l'étape a) peut être obtenue par oxydation superficielle du métal de la paroi sur une fraction de l'épaisseur de cette paroi, ou bien par dépôt en surface de la paroi d'une couche additionnelle d'un matériau électriquement isolant.

La gorge non traversante de contour fermé formée à l'étape b) peut être obtenue par une technique du groupe formé par : attaque sélective chimique, attaque sélective physique, gravure laser, sciage de précision et les combinaisons de ces techniques.

Le procédé peut également comprendre une étape de : d) soudage de conducteurs électriques sur les plages de contact électrique externe et interne, de manière à former ladite liaison électrique.

Le procédé peut également comprendre une étape d'injection d'un matériau de remplissage électriquement isolant dans la gorge non traversante de contour fermé formée à l'étape b).

Les étapes a) à c) peuvent être exécutées directement sur la paroi du boîtier du dispositif médical actif, ou bien sur une pièce indépendante du boîtier du dispositif médical actif, le procédé comprenant une étape ultérieure de report et de solidarisation de cette pièce indépendante sur le reste du boîtier du dispositif médical actif.

L'invention vise également un boîtier de dispositif médical actif réalisé selon les enseignements du procédé ci-dessus

Plus précisément, un tel boîtier comprend une paroi métallique présentant un côté externe et un côté interne et supportant au moins une traversée hermétique et électriquement isolante pour le passage d'une liaison électrique au travers de cette paroi métallique.

Côté interne, la paroi comprend : une couche interne électriquement isolante ; une gorge non traversante de contour fermé délimitant dans la paroi un îlot métallique physiquement et électriquement isolé du reste de la paroi, cette gorge s'étendant sur la totalité de l'épaisseur de la couche interne électriquement isolante et de celle de la paroi, en laissant intacte l'épaisseur de la couche externe électriquement isolante ; et une plage de contact électrique à l'îlot métallique, la couche interne électriquement isolante étant absente dans la région de cette plage de contact électrique.

Côté externe, la paroi comprend : une couche externe électriquement isolante ; et une plage de contact électrique à l'îlot métallique, la couche externe électriquement isolante étant absente dans la région de cette plage de contact électrique, et la gorge côté interne laissant intacte l'épaisseur de la couche externe électriquement isolante de manière à faire supporter mécaniquement l'îlot métallique par cette couche externe.

Un matériau de remplissage électriquement isolant peut éventuellement garnir la gorge non traversante de contour fermé.

La paroi peut être une paroi d'une seule pièce du boîtier du dispositif médical actif, ou bien celle d'une pièce indépendante rapportée sur le reste du boîtier du dispositif médical actif.

On va maintenant décrire un exemple de mise en oeuvre du dispositif de l'invention, en référence aux dessins annexés où les mêmes références numériques désignent d'une figure à l'autre des éléments identiques ou fonctionnellement semblables.
La Figure 1 est une vue en élévation, en coupe, d'une traversée selon l'état de la technique.
La Figure 2 illustre les étapes successives de réalisation d'une traversée selon l'invention.
La Figure 3 est une vue générale en élévation d'un boîtier de dispositif pourvu d'une traversée selon l'invention.
La Figure 4 est une vue en coupe du détail repéré IV-IV sur la Figure 3, au niveau de la traversée.
La Figure 5 est une vue perspective, de dessous, d'une variante de l'invention où la traversée est réalisée sur une pièce métallique ultérieurement rapportée sur le boîtier du dispositif.
La Figure 6 est une vue perspective de dessus de la variante de la Figure 5, avec la pièce rapportée soudée au boîtier.

On va maintenant décrire un exemple de réalisation du dispositif de l'invention.

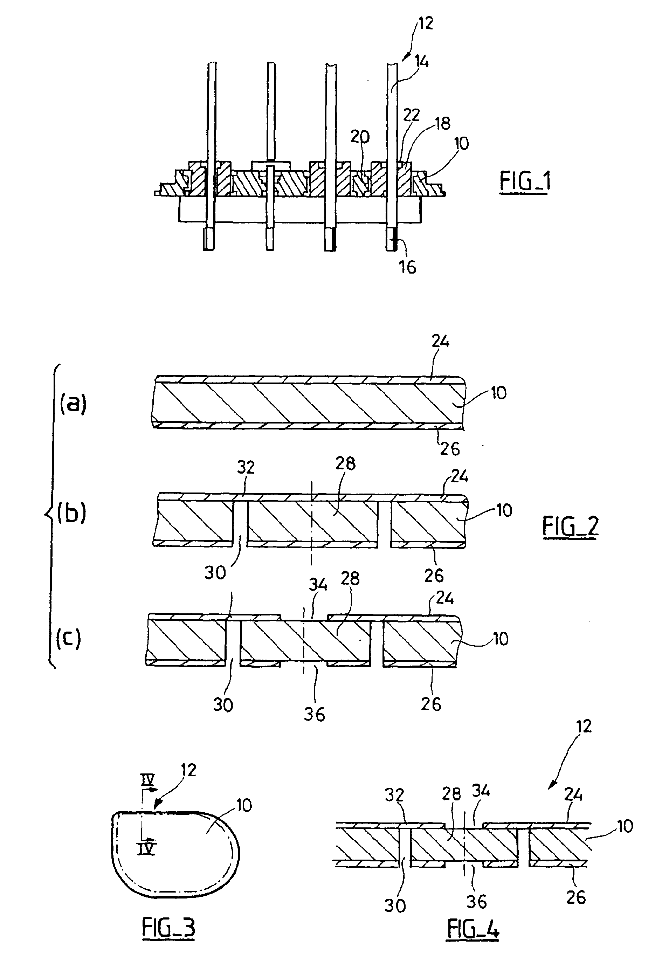

La Figure 1 illustre une traversée selon l'état de la technique. La référence 10 désigne le boîtier métallique en titane du générateur (ou une pièce métallique en titane qui sera ultérieurement rapportée sur ce boîtier), avec une pluralité de picots de traversée 12 dont une extrémité 14 débouche côté extérieur du boîtier pour liaison à un organe de contact de la tête de connecteur après report de cette dernière sur le boîtier, et l'extrémité opposée 16 est destinée à être reliée à des fils de contact assurant une liaison avec les circuits internes du générateur. Ces picots 10 sont généralement réalisés en platine/iridium, et ils sont isolés du boîtier 10 par un élément 18 en céramique ou en verre. Chaque élément isolant 18 est cerclé d'une collerette métallique 20 permettant d'assurer une solidarisation hermétique au boîtier en titane 10 par une brasure à l'or. Les picots 12 sont également solidarisés en 22 à l'élément isolant 18 par une brasure à l'or.

La Figure 2 illustre les étapes de réalisation d'une traversée selon l'invention.

Essentiellement, l'invention propose de s'affranchir de toute pièce intermédiaire (telle que l'élément isolant céramique 18 des traversées conventionnelles) et de réserver dans l'épaisseur du boîtier même une zone isolée qui servira à assurer la conduction électrique. La réalisation de cette zone, ci-après désignée "îlot" nécessite toutefois la prise en compte d'un certain nombre de contraintes, notamment :
- la faible épaisseur du boîtier en titane, qui est de 300 µm environ ;
- la biocompatibilité et la biostabilité du boîtier, ainsi que la résistance à la corrosion ;
- l'absence de trop grande fragilité dans la région de la traversée, car cette région sera probablement manipulée par des opératrices lors du montage du dispositif ;
- la compatibilité de la nouvelle technique avec les étapes d'assemblage du dispositif en amont et en aval de la chaîne de production ;
- la résistance aux différents procédés utilisés tels que soudage laser et collage de la tête de connecteur.

À cet effet, la technique de l'invention prévoit, dans une première étape illustrée Figure 2a, de créer de part et d'autre de la paroi du boîtier en titane 10 une couche isolante, avec une couche isolante externe 24 et une couche isolante interne 26. Ces couches pourront être réalisées par oxydation du titane du boîtier 10 sur une profondeur contrôlée, ou par dépôt d'une couche de matériau isolant, par exemple de dioxyde de silicium en surface de l'épaisseur 10 du boîtier en titane. L'épaisseur de chacune des couches 24, 26 est par exemple de l'ordre de 10 µm, pour une épaisseur de boîtier de l'ordre de 300 µm.

Ces couches 24 et 26 peuvent être réalisées par exemple par oxydation thermique, oxydation ou tout autre procédé tel qu'oxydation plasma ou dépôt chimique. Il est possible de procéder par anodisation, en soumettant le boîtier à une différence de potentiel et en le mettant simultanément en contact avec une solution d'eau et d'acide sulfurique, soit par trempage soit à l'aide d'un pinceau-électrode.

L'étape suivante, illustrée Figure 2b, consiste à ménager un îlot conducteur 28 physiquement isolé dans l'épaisseur du boîtier par creusement d'une gorge non traversante 30.

Dans le sens de la profondeur, la gorge 30 est creusée depuis le côté interne sur toute l'épaisseur de la couche isolante interne 26 et sur la totalité de l'épaisseur de la paroi du boîtier métallique 10. En revanche, la couche externe 24 est laissée intacte, de manière que l'îlot 28 puisse être supporté mécaniquement par le pont 32 formé par cette couche externe entre la zone isolée 28 et le reste de la couche métallique du boîtier 10.

La couche externe 24 forme en outre une barrière hermétique entre l'intérieur du boîtier et l'environnement extérieur.

Dans le plan de la surface du boîtier, la gorge 30 est creusée sur un contour fermé, de manière à complètement isoler électriquement l'îlot 28 du reste du boîtier 10 sur toute sa périphérie, l'îlot restant en revanche mécaniquement relié au reste du boîtier 10 par les ponts 32 de la couche isolante externe 24 formée à l'étape précédente.

Le creusement de la gorge 30 peut être réalisé par divers procédés en eux-mêmes connus.

Ce creusement peut être effectué par gravure sélective chimique (interactions d' espèces réactives avec le titane) ou physique (bombardement par des ions), ou encore par tout procédé de micro-structuration tel que le sciage de précision.

On pourra utiliser en particulier les techniques connues de gravure plasma (gravure par ions réactifs GIR ou RIE, *Reactive Ion Etching*) ou les procédés dits TIDE (*Ttitanium Inductively-coupled plasma Deep Etching*) issus des technologies de réalisation des semi-conducteurs et des microstructures électromécaniques MEMS. Il est également possible de procéder par gravure laser, ou encore par gravure chimique en trempant le titane ou la couche isolante dans une solution chimique acide ou basique (selon le matériau à graver), en protégeant au préalable la zone non-attaquée par un masque sélectif.

Ces procédés peuvent être également combinés entre eux pour minimiser le temps de découpe du titane.

L'étape suivante, illustrée Figure 2c, consiste à réaliser sélectivement des ouvertures 34, 36 dans les couches isolantes respectives 24, 26 de manière à mettre à nu des zones sur lesquelles pourront être brasés des fils ou des picots pour assurer la prise de contact électrique avec l'îlot conducteur central 28.

Ces ouvertures 34, 36 peuvent être réalisées par attaque chimique sélective, par exemple par gravure ionique réactive (GIR ou RIE). Les dimensions seront choisies de manière à permettre la réalisation aisée du contact avec le picot ou le conducteur électrique, en prévoyant toutefois de laisser subsister sur la face externe suffisamment de matériau de la couche externe 24 pour ne pas fragiliser la structure dans la région du pont de matière 32 qui supporte mécaniquement l'îlot 28.

Optionnellement, le vide correspondant à la gorge 30 peut être rempli d'un matériau de garnissage électriquement isolant tel qu'une colle ou une résine telles que celles utilisées dans les puces électroniques, qui aura pour fonction de : pallier toute fragilité du matériau résultant des différentes attaques qui ont été effectuées, procurer un isolement électrique supplémentaire de l'îlot central 28 et enfin accroître l'herméticité générale de la structure.

Les Figures 3 et 4 montrent le résultat obtenu, dans le cas d'un mode de réalisation où le procédé des Figures 2a à 2c a été mis en oeuvre directement sur le corps du boîtier 10 proprement dit (ou d'une partie du corps de ce boîtier, par exemple un demi-boîtier).

Dans ce cas, puisque le boîtier intègre directement la traversée 12, on s'affranchit des étapes d'assemblage de la traversée qui étaient nécessaires auparavant, avec réduction significative du coût de fabrication et du temps de cycle du produit.

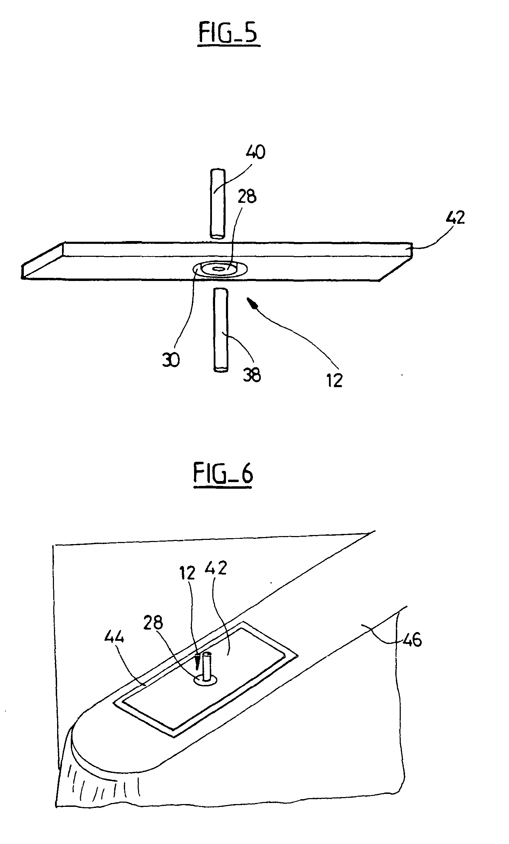

Les Figures 5 et 6 illustrent une variante de réalisation dans laquelle la traversée est réalisée sur une pièce distincte, ultérieurement rapportée sur le reste du boîtier.

Sur la Figure 5, on a représenté la traversée 12, qui présente une structure identique à celle de la Figure 4, avec deux picots intérieur 38 et extérieur 40 prêts à être reliés par brasure à l'îlot central isolé 28. La différence réside dans le fait que la traversée 12 n'est pas réalisée sur le corps du boîtier proprement dit, mais sur une platine de titane 42 qui sera ensuite intégrée au reste du boîtier 46 et solidarisée à celui-ci par une brasure périphérique 46.

Cette variante facilite l'industrialisation du procédé, dans la mesure où il est plus aisé de réaliser la traversée sur des pièces plates de petites dimensions telles que la platine 42 que directement sur le corps d'un boîtier.

Elle implique certes la réalisation d'une soudure supplémentaire (soudure 44), mais il s'agit là d'une soudure titane/titane, donc entre deux matériaux identiques, qui n'est pas sujette aux problèmes de différence de coefficients de dilatation thermique rencontrés avec les soudures céramique/ titane des traversées actuelles.

## Revendications

1. Un procédé de réalisation d'une traversée hermétique et électriquement isolante, pour le passage d'une liaison électrique au travers d'une paroi métallique électriquement conductrice (10) d'un dispositif électronique, notamment d'un dispositif médical actif, cette paroi présentant un côté externe et un côté interne, procédé comportant une étape de :
a) formation d'une couche électriquement isolante (24, 26) sur chacune des faces externe et interne de la paroi (10) ; et **caractérisé en outre par** les étapes suivantes :
b) coté interne de la paroi, formation d'une gorge non traversante de contour fermé (30) délimitant dans la paroi un îlot métallique (28) physiquement et électriquement isolé du reste de la paroi, par creusement de la totalité de l'épaisseur de la couche interne électriquement isolante (26) et de celle de la paroi (10), en laissant intacte l'épaisseur de la couche externe électriquement isolante (24) de manière à faire supporter mécaniquement l'îlot métallique par cette couche externe ; et
c) mise à nu côté externe, et respectivement côté interne, d'une plage (34, 36) de contact électrique à l'îlot métallique, par enlèvement localisé de matière sur l'épaisseur de la couche externe, respectivement interne, électriquement isolante (24, 26).

2. Le procédé de la revendication 1, dans lequel la couche électriquement isolante (24, 26) formée à l'étape a) est obtenue par oxydation superficielle du métal de la paroi (10) sur une fraction de l'épaisseur de cette paroi.

3. Le procédé de la revendication 1, dans lequel la couche électriquement isolante (24, 26) formée à l'étape a) est obtenue par dépôt en surface de la paroi d'une couche additionnelle d'un matériau électriquement isolant.

4. Le procédé de la revendication 1, dans lequel la gorge non traversante de contour fermé (30) formée à l'étape b) est obtenue par une technique du groupe formé par : attaque sélective chimique, attaque sélective physique, gravure laser, sciage de précision et les combinaisons de ces techniques.

5. Le procédé de la revendication 1, comprenant en outre une étape de :
d) soudage de conducteurs électriques (38, 40) sur les plages de contact électrique externe (34) et interne (36), de manière à former ladite liaison électrique.

6. Le procédé de la revendication 1, comprenant en outre une étape d'injection d'un matériau de remplissage électriquement isolant dans la gorge non traversante de contour fermé (30) formée à l'étape b).

7. Le procédé de la revendication 1, dans lequel les étapes a) à c) sont exécutées directement sur la paroi (10) du boîtier du dispositif médical actif.

8. Le procédé de la revendication 1, dans lequel les étapes a) à c) sont exécutées sur une pièce (42) indépendante du boîtier du dispositif médical actif, le procédé comprenant une étape ultérieure de report et de solidarisation de cette pièce indépendante (42) sur le reste du boîtier (46) du dispositif médical actif.

9. Un boîtier de dispositif électronique, notamment de dispositif médical actif, comprenant une paroi métallique (10) présentant un côté externe et un côté interne et supportant au moins une traversée hermétique et électriquement isolante pour le passage d'une liaison électrique au travers de cette paroi métallique ladite paroi comprenant côté interne une couche interne électriquement isolante (26) et coté externe une couche externe électriquement isolante (24) **caractérisé en ce que** ladite paroi comprend en outre :
- côté interne de la paroi :
• une gorge non traversante de contour fermé (30) délimitant dans la paroi un îlot métallique (28) physiquement et électriquement isolé du reste de la paroi, cette gorge s'étendant sur la totalité de l'épaisseur de la couche interne électriquement isolante (26) et de celle de la paroi (10) ; et
• une plage (36) de contact électrique à l'îlot métallique, la couche interne électriquement isolante (24) étant absente dans la région de cette plage de contact électrique, et
- côté externe de la paroi :
• une plage (34) de contact électrique à l'îlot métallique, la couche externe électriquement isolante (24) étant absente dans la région de cette plage de contact électrique,
la gorge (30) côté interne laissant intacte l'épaisseur de la couche externe électriquement isolante (24) de manière à faire supporter mécaniquement l'îlot métallique par cette couche externe.

10. Le boîtier de la revendication 9, comprenant un matériau de remplissage électriquement isolant dans la gorge non traversante de contour fermé (30).

11. Le boîtier de la revendication 9, dans lequel ladite paroi (10) est une paroi d'une seule pièce du boîtier du dispositif médical actif.

12. Le boîtier de la revendication 9, dans lequel ladite paroi (10) est la paroi d'une pièce indépendante (42) rapportée sur le reste du boîtier (46) du dispositif médical actif.

## Claims

1. Method for producing a hermetic and electrically insulating feed-through for passing an electrical connection through an electrically conductive metal wall (10) of an electronic device, in particular an active medical device, this wall having an external side and an internal side, which method comprises a step of:
a) forming an electrically insulating layer (24, 26) on each of the faces, external and internal, of the wall (10); and is furthermore **characterized by** the following steps:
b) on the internal side of the wall, forming a groove (30) which does not pass fully through and has a closed contour delimiting a metal island (28) in the wall, which is physically and electrically insulated from the rest of the wall, by excavating the entire thickness of the internal electrically insulating layer (26) and the entire thickness of the wall (10), leaving intact the thickness of the external electrically insulating layer (24) so that the metal island is mechanically supported by this external layer; and
c) on the external side, and respectively on the internal side, exposing an area (34, 36) for electrical contact with the metal island, by localized removal of material over the thickness of the external, and respectively internal, electrically insulating layer (24, 26).

2. Method according to Claim 1, wherein the electrically insulating layer (24, 26) formed in step a) is obtained by surface oxidation of the metal of the wall (10) over a fraction of the thickness of this wall.

3. Method according to Claim 1, wherein the electrically insulating layer (24, 26) formed in step a) is obtained by depositing an additional layer of an electrically insulating material on the surface of the wall.

4. Method according to Claim 1, wherein the groove, (30), which does not pass fully through and has a closed contour, formed in step b) is obtained by a technique selected from the group consisting of: selective chemical attack, selective physical attack, laser etching, precision sawing and combinations of these techniques.

5. Method according to Claim 1, furthermore comprising a step of:
d) welding electrical conductors (38, 40) onto the external (34) and internal (36) electrical contact areas, so as to form the said electrical connection.

6. Method according to Claim 1, furthermore comprising a step of injecting an electrically insulating filler material into the groove (30), which does not pass fully through and has a closed contour, formed in step b).

7. Method according to Claim 1, wherein steps a) to c) are carried out directly on the wall (10) of the housing of the active medical device.

8. Method according to Claim 1, wherein steps a) to c) are carried out on an independent part (42) of the housing of the active medical device, the method comprising a subsequent step of attaching and securing this independent part (42) to the rest of the housing (46) of the active medical device.

9. Housing of an electronic device, in particular an active medical device, comprising a metal wall (10) having an external side and an internal side and bearing at least one hermetic and electrically insulating feed-through for passing an electrical connection through this metal wall, the said wall comprising an internal electrically insulating layer (26) on the internal side and an external electrically insulating layer (24) on the external side, **characterized in that** the said wall furthermore comprises:
- on the internal side of the wall:
• a groove (30) which does not pass fully through and has a closed contour delimiting a metal island (28) in the wall, which is physically and electrically insulated from the rest of the wall, this groove extending over the entire thickness of the internal electrically insulating layer (26) and the entire thickness of the wall (10); and
• an area (36) for electrical contact with the metal island, the internal electrically insulating layer (26) being absent in the region of this electrical contact area, and
- on the external side of the wall:
• an area (34) for electrical contact with the metal island, the external electrically insulating layer (24) being absent in the region of this electrical contact area,
the groove (30) on the internal side leaving the thickness of the external electrically insulating layer (24) intact so that the metal island is mechanically supported by this external layer.

10. Housing according to Claim 9, comprising an electrically insulating filler material in the groove (30) which does not pass fully through and has a closed contour.

11. Housing according to Claim 9, wherein the said wall (10) is a wall integral with the housing of the active medical device.

12. Housing according to Claim 9, wherein the said wall (10) is a wall of an independent part (42) attached to the rest of the housing (46) of the active medical device.

## Patentansprüche

1. Verfahren für die Herstellung einer hermetischen und elektrisch isolierenden Durchführung für die Durchführung einer elektrischen Verbindung durch eine elektrisch leitende Wand (10) einer elektronischen Vorrichtung, insbesondere einer aktiven medizinischen Vorrichtung, wobei diese Wand eine Außenseite und eine Innenseite aufweist, wobei das Verfahren den folgenden Schritt umfasst:
a) Bilden jeweils einer elektrisch isolierenden Schicht (24, 26) auf der äußeren Fläche und auf der inneren Fläche der Wand (10); und außerdem **gekennzeichnet durch** die folgenden Schritte:
b) auf der Innenseite der Wand Bilden einer Nichtdurchgangsnut mit geschlossenem Umriss (30), die in der Wand eine vom Rest der Wand physikalisch und elektrisch isolierte Metallinsel (28) begrenzt, **durch** Aushöhlen der gesamten Dicke der elektrisch isolierenden inneren Schicht (26) und jener der Wand (10), während die Dicke der elektrisch isolierenden äußeren Schicht (24) intakt gelassen wird, derart, dass die metallische Insel von dieser äußeren Schicht mechanisch getragen wird; und
c) auf der Außenseite bzw. auf der Innenseite Freilegen eines elektrischen Kontaktbereichs (34, 36) mit der metallischen Insel **durch** räumlich begrenzte Materialabtragung auf der Dicke der elektrisch isolierenden äußeren bzw. inneren Schicht (24, 26).

2. Verfahren nach Anspruch 1, wobei die elektrisch isolierende Schicht (24, 26), die im Schritt a) gebildet wird, durch Oberflächenoxidation des Metalls der Wand (10) in einem Bruchteil der Dicke dieser Wand erhalten wird.

3. Verfahren nach Anspruch 1, wobei die elektrisch isolierende Schicht (24, 26), die im Schritt a) gebildet wird, durch Abscheidung auf der Oberfläche der Wand einer zusätzlichen Schicht aus einem elektrisch isolierenden Material erhalten wird.

4. Verfahren nach Anspruch 1, wobei die Nichtdurchgangsnut mit geschlossenem Umriss (30), die im Schritt b) gebildet wird, durch eine Gruppentechnik erhalten wird, die gebildet ist aus: selektivem chemischen Angriff, selektivem physikalischen Angriff, Laserätzung, Präzisionssägen und Kombinationen dieser Techniken.

5. Verfahren nach Anspruch 1, das außerdem den folgenden Schritt umfasst:
d) Löten elektrischer Leiter (38, 40) auf die äußeren (34) und inneren (36) elektrischen Kontaktbereiche in der Weise, dass die elektrische Verbindung gebildet wird.

6. Verfahren nach Anspruch 1, das außerdem einen Schritt des Einleitens eines elektrisch isolierenden Füllmaterials in die Nichtdurchgangsnut mit geschlossenem Umriss (30), die im Schritt b) gebildet wird, umfasst.

7. Verfahren nach Anspruch 1, wobei die Schritte a) bis c) direkt auf der Wand (10) des Gehäuses der aktiven medizinischen Vorrichtung ausgeführt werden.

8. Verfahren nach Anspruch 1, wobei die Schritte a) bis c) an einem von dem Gehäuse der aktiven medizinischen Vorrichtung unabhängigen Teil (42) ausgeführt werden, wobei das Verfahren einen späteren Schritt des Übertragens und Befestigens dieses unabhängigen Teils (42) zu bzw. an dem Rest des Gehäuses (46) der aktiven medizinischen Vorrichtung umfasst.

9. Gehäuse einer elektronischen Vorrichtung, insbesondere einer aktiven medizinischen Vorrichtung, das eine Metallwand (10) umfasst, die eine äußere Seite und eine innere Seite aufweist und wenigstens eine hermetische und elektrisch isolierende Durchführung für den Durchgang einer elektrischen Verbindung durch diese Metallwand trägt, wobei die Wand auf der Innenseite eine elektrisch isolierende innere Schicht (26) und auf der äußeren Seite eine elektrisch isolierende äußere Schicht (24) umfasst, **dadurch gekennzeichnet, dass** die Wand außerdem Folgendes umfasst:
- auf der Innenseite der Wand:
- eine Nichtdurchgangsnut mit geschlossenem Umriss (30), die in der Wand eine vom Rest der Wand physikalisch und elektrisch isolierte Metallinsel (28) begrenzt, wobei sich diese Nut über die gesamte Dicke der elektrisch isolierenden inneren Schicht (26) und jene der Wand (10) erstreckt; und
- einen Bereich (36) für den elektrischen Kontakt mit der Metallinsel, wobei die elektrisch isolierende innere Schicht (26) in der Zone dieses Bereichs für elektrischen Kontakt fehlt, und
- auf der Außenseite der Wand:
- einen Bereich (34) für elektrischen Kontakt mit der Metallinsel, wobei die elektrisch isolierende äußere Schicht (24) in der Zone dieses Bereichs für elektrischen Kontakt fehlt,
wobei die Nut (30) auf der Innenseite die Dicke der elektrisch isolierenden äußeren Schicht (24) intakt lässt, derart, dass die Metallinsel von dieser äußeren Schicht mechanisch getragen wird.

10. Gehäuse nach Anspruch 9, das ein elektrisch isolierendes Füllmaterial in der Nichtdurchgangsnut mit geschlossenem Umriss (30) aufweist.

11. Gehäuse nach Anspruch 9, wobei die Wand (10) eine einteilig mit dem Gehäuse der aktiven medizinischen Vorrichtung ausgebildete Wand ist.

12. Gehäuse nach Anspruch 9, wobei die Wand (10) die Wand eines unabhängigen Teils (42) ist, die an den Rest des Gehäuses (46) der aktiven medizinischen Vorrichtung angefügt ist.
